# EUROPEAN PATENT SPECIFICATION

(11) **EP 1 532 992 B1**
(45) Date of publication and mention of the grant of the patent: **15.09.2010**
(21) Application number: 03794052.5
(22) Date of filing: 06.02.2003
(51) Int. Cl.: A61M 1/06, F16K 15/14

(54) **MANUAL BREAST PUMP**
MANUELLE BRUSTPUMPE
DISPOSITIF MANUEL D'EXTRACTION DE LAIT

(30) Priority: 29.08.2002 JP 2002251463
(43) Date of publication of application: 25.05.2005
(73) Proprietor: Pigeon Corporation, Chuo-ku Tokyo (JP)
(72) Inventor: TASHIRO, Mitsuo, Chiyoda-ku, Tokyo 101-0043 (JP); UEHARA, Hiroyuki, Chiyoda-ku, Tokyo 101-0043 (JP); ODABAYASHI, Yuko, Chiyoda-ku, Tokyo 101-0043 (JP)
(74) Representative: Kremer, Simon Mark
(86) International application number: PCT/JP2003/001223
(87) International publication number: WO 2004/022134

(56) References cited:
- WO-A-01/34226
- WO-A-2004/014462
- WO-A1-01/34226
- GB-A- 2 166 353
- JP-A- 11 178 917
- JP-B2- 63 067 071
- JP-U- 61 089 561
- JP-Y2- 7 041 468
- JP-Z1- 33 399
- JP-Z1- 33 399
- US-A- 4 583 970

## Description

### FIELD OF THE INVENTION

This invention relates to an improvement of a manual breast pump, for example.

### BACKGROUND OF THE INVENTION

As a manual breast pump, there is one that was proposed by the applicant of the present patent application and illustrated in FIG. 13 of the accompanying drawings (Jpn. Pat. Appln. Laid-Open Publication Hei. 1-190364).

The manual breast pump 1 comprises a main body 5 thereof that is provided with a suction portion 2 and can be detachably fitted to a bottle 6. A vent pipe 3, which may typically be a rubber pipe, extends from the breast pump main body 5. A negative pressure generating means 4, which is a hollow rubber member, is fitted to the opposite end of the vent pipe 3.

The user holds the bottle 6 with one of his or her hands and fit the horn-shaped suction portion 2 to a breast at the opening thereof. As the user repeatedly grasp the negative pressure generating means 4 with the other hand to generate negative pressure, which is applied to the suction portion 2 by way of the vent pipe 3 and the breast pump main body 5, mother's milk is sucked and falls into the bottle 2.

By the way, such a manual breast pump 1 is difficult to use because it needs to be operated by two hands. Additionally, although the bottle 2 and the vent pipe 3 can be removed from the breast pump main body 5, the user can dismantle neither the breast pump main body 5 that has a number of branch passages nor the negative pressure generating means 4. So, it is difficult for the user to routinely clean the inside of the breast pump main body 5.

Thus, while the manual breast pump 1 needs to be held in a hygienically clean condition because it deals with mother's milk to be ingested by infants, it requires cumbersome maintance operations.

WO 01/34226 A describes a milk sucking pump (1) having a detachable breast joining piece (6) and a hand-pump unit (3). The hand pump unit has a cap element (5) and a pump-piston (7) which can be moved in a piston swept volume (5.2) by a rotatable actuating handle (4) that has a return mechanism. The cap element (5) and the piston-swept volume (5.2) are combined to form a uniform cap component.

JP 00033399 Z1 describes a pump having a syringe like mechanism (6, 3) connected via a tube (5) to a tubular element (1) having a flared end (2) and a bulbous portion (7).

GB-A-2 166353 A describes a single handed breast pump comprising a pump body (1) housing a piston (7) releasably connected to a hand lever (9) itself releasably pivoted to the pump body (1), the latter including a funnel (2) and an outlet (4). The funnel connects with a variable volume chamber in the pump body, said chamber having valves (11, 24).

US-A-4 583 970 describes a milk suction device consisting of a funnel and a piston pump connected thereto. A hollow piston rod is constructed as an outwardly guided aeration duct and has a return valve as its opening located in the suction space of a cylinder of the pump. A ventilation valve is arranged in a front face of the pump cylinder opposite the piston rod.

In view of the above identified circumstances, it is therefore the object of the present invention to provide a manual breast pump that can be easily dismantled for cleaning and other purposes, cleaned with ease and handled in a hygienically clean condition.

### DISCLOSURE OF THE INVENTION

According to the present invention, the above object is achieved by providing a manual breast pump according to claim 1.

Preferred embodiments may be found in the dependent claims.

### BRIEF DESCRIPION OF THE DRAWINGS

FIG. 1 is a schematic perspective view of the first embodiment of breast pump according to the invention.
FIG. 2 is a schematic cross sectional view of the embodiment of breast pump of FIG. 1.
FIG. 3 is a schematic partial cross sectional view of the embodiment of breast pump of FIG. 1, illustrating the structure of the rotative and supportive shaft for securing the lever to the breast pump.
FIG. 4 is a schematic illustration of the embodiment of the breast pump of FIG. 1, illustrating the structure of the engagement at the rotative and supportive shaft of the lever.
FIG. 5 is a schematic lateral view of the embodiment of breast pump of FIG. 1.
FIG. 6 is a schematic cross sectional view of the embodiment of breast pump of FIG. 1 when the embodiment is operated for sucking milk.
FIG. 7 is a schematic cross sectional view of the embodiment of breast pump of FIG. 1 when the embodiment is operated for leaking air.
FIG. 8 is a schematic perspective view of the valve main body of the pressure regulating means arranged on the outer surface of the sleeve of the embodiment of breast pump of FIG. 1, the valve main body being part of the valve structure.
FIG. 9 is a schematic perspective view of the valve box that is combined with the valve main body, which is part of the valve structure, to form the pressure regulating means that is arranged on the outer surface of the sleeve of the embodiment of breast pump of FIG. 1.
FIG. 10 is a schematic illustration of operation of the pressure regulating means that is arranged on the outer surface of the sleeve of the embodiment of breast pump of FIG. 1.
FIG. 11 is another schematic illustration of operation of the pressure regulating means that is arranged on the outer surface of the sleeve of the embodiment of breast pump of FIG. 1.
FIG. 12 is still another schematic illustration of operation of the pressure regulating means that is arranged on the outer surface of the sleeve of the embodiment of breast pump of FIG. 1.

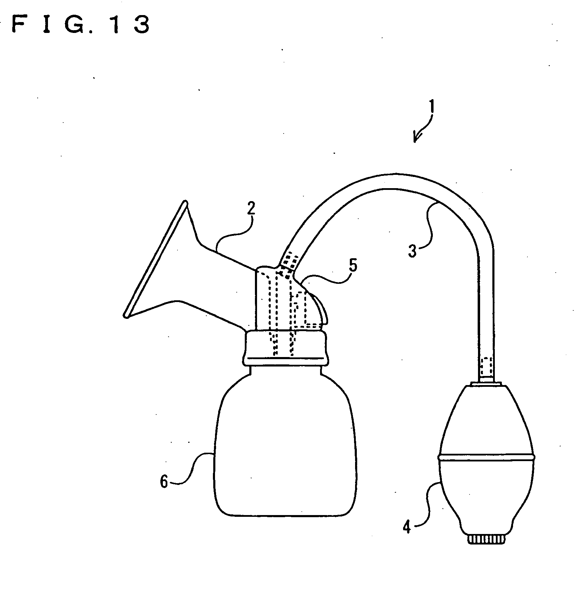

FIG. 13 is a schematic illustration of a known breast pump.

### BEST MODE FOR CARRYING OUT THE INVENTION

The present invention will be described in detail by referring to the accompanying drawings hereinafter.

The following embodiments includes various preferable technical limitations because they are preferred concrete embodiments of invention. However, the scope of the present invention is not limited to them unless there is a description to limit to it.

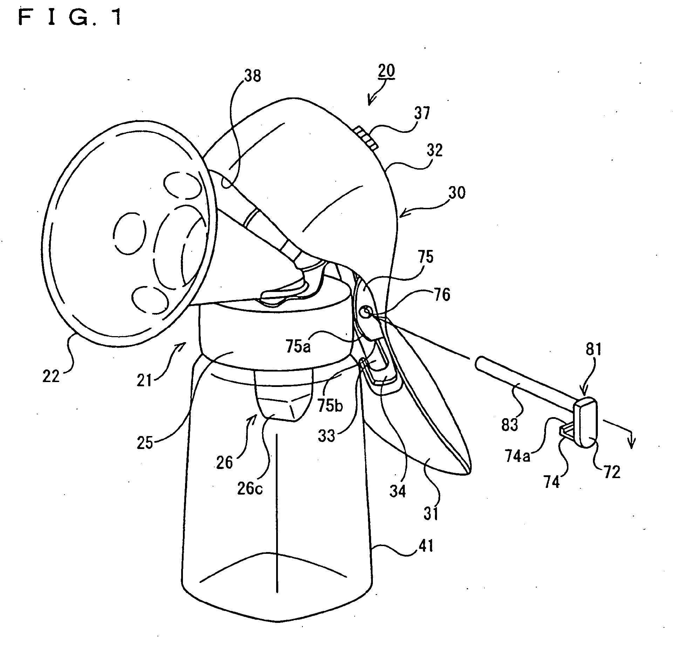

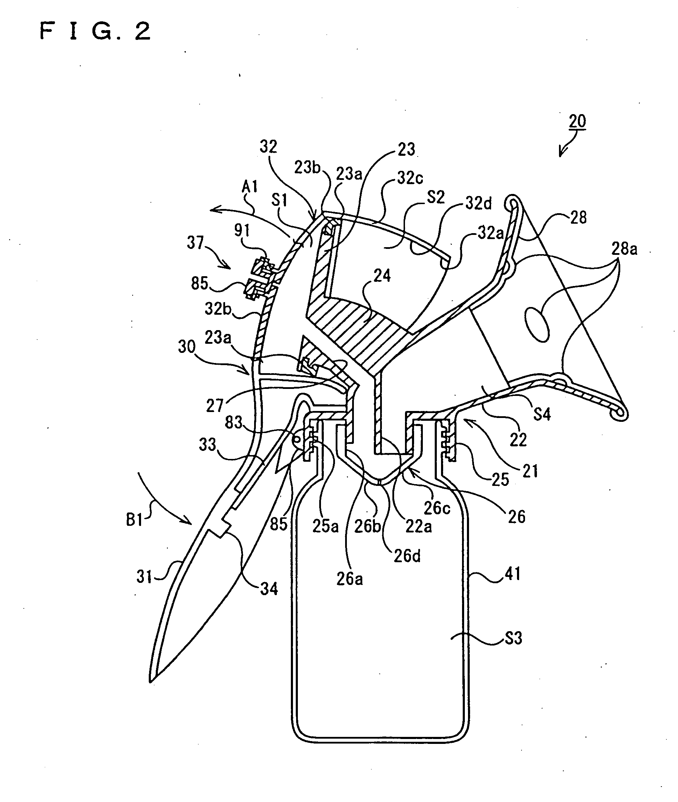

FIG. 1 is a schematic perspective view of an embodiment of breast pump 20 according to the invention, illustrating its overall configuration. FIG. 2 is a schematic cross sectional view of the embodiment. As shown in FIGS. 1 and 2, the breast pump 20 comprises a breast pump main body 21 (to be referred to simply as "main body" hereinafter) and a lever 30 (to be also referred to as "grip" or "handle"), wherein the lever 30 can be attached and detached to the breast pump main body 21.

The main body 21 is made of a synthetic resin material that is relatively light, tough and easily moldable. Synthetic resin materials that can be used for the main body 21 include polycarbonate, polycycloolefin and polyethersulfone and the like. The main body 21 comprises a detachable connecting portion 25 to attach to and detach from a bottle 41 for storing sucked milk. As shown in FIG. 2, the detachable connecting portion 25 has a flat and cylindrical profile and is typically provided with an internal thread 25a so that it may be engaged with the external thread formed around the neck of the bottle 41. The bottle 41 may be one that is dedicated to the breast pump 20 or any other one that can be engaged with the detachable connecting portion 25.

A conical or horn-shaped suction portion 22 inclined with its opening facing the outside are arranged at the upper portion of the detachable connecting portion 25 of the main body 21. A buffer 28 made of an elastic material such as silicon, elastomer or natural rubber is detachably fitted to the suction portion 22 at a position close to the open end of the suction portion. The buffer 28 portion reduces the stimulus given to the breast touched by suction portion 22 during the sucking operation so that the breast may be free from pain. The buffer 28 portion has a number of projections 28a on the inner peripheral surface thereof. The suction portion 22 has vent passage 22a, which allows both air and sucked milk to pass through it and is bent downward and directed toward the bottle 41. The opening and its periphery of the suction portion vent passage 22a of the suction portion 22 are located inside the main body 21 and the detachable connecting portion, fit with a cellular valve 26. Another vent passage 27 is provided adjacent to the suction portion vent passage 22a. As shown in FIG. 2, the bottom end opening of the vent passage 27 is communicating with the suction portion vent passage 22a within the cell 26a of the cellular valve 26 and the vent passage 27 runs upward through piston rod 24 and terminates at the top end opening. The lower end of the suction portion vent passage is lengthened than the lower end of the vent passage 27 so that sucked milk can hardly enter the vent passage 27.

As seen in FIGS. 1 and 2, the cellular valve 26 has a cap-like profile as a whole and is made of an elastic material such as silicon, elastomer or natural rubber. The oppositely disposed elastic lateral walls 26b, 26c of the cellular valve 26 are inclined so as to come closer to each other as they approach the lower ends thereof. Slit 26d is constructed at the bottom end where both wall 26b and 26c come closely. Thus, when the volume of sucked milk stored in the cell 26a gets to a predetermined amount, the weight on the change of the negative pressure when negative pressure released as described later make leading edge of the lateral walls 26b, 26c open to allow milk to fall into the bottle 41. Due to the slit 26d constructed at the lower ends of the inclined lateral walls, no air gets into the cell 26a from the bottle 41 when negative pressure prevails in the inside.

The main body 21 has a fixed piston 23 that is arranged at the top of and integral with the above described piston rod 24. The fixed piston 23 is arranged transversally at the top of the piston rod 24 in a direction opposite to the direction in which the suction portion 22 extends. The fixed piston 23 has a disk-shaped or an elliptic profile.

In FIG. 2, the lever 30 has a shape prolonged approximately upward and downward and is provided at an end thereof, or the top end in FIG. 2, with a sleeve 32, which is integral with the lever 30 and has an opening facing the above described fixed piston 23. The lever 30 is a molded product and as a whole made of relatively lightweight synthetic resin such as polycarbonate, polycycloolefin or polyethersulfone.

The other end of the lever 30 has a handle-like profile which extends downward as shown in FIG. 2 to form a lever main body 31 as operating part.

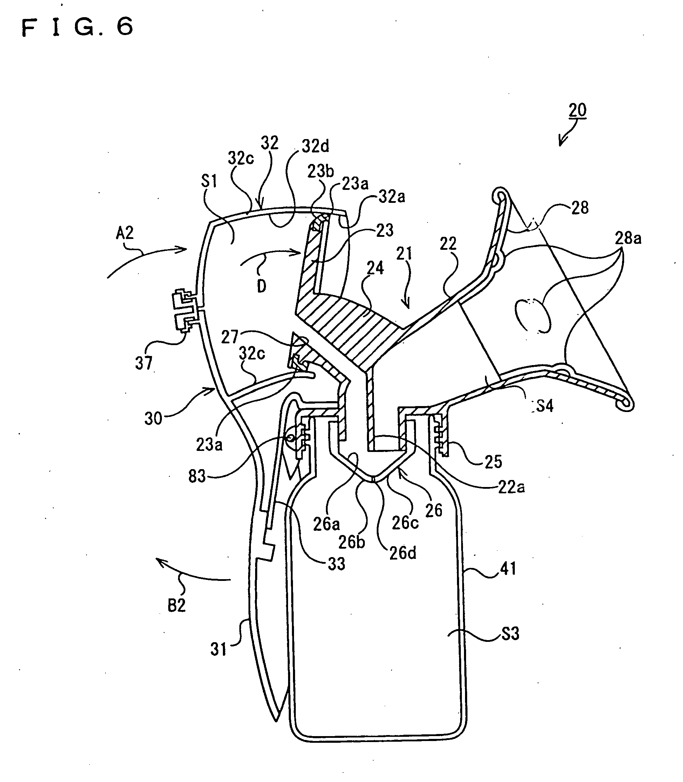

The lever 30 can be attached to and detached from the main body 21 in a manner as will be described hereinafter. When the lever 30 is fixed to its position, it is secured to the main body 21 at a longitudinal middle position thereof by means of a rotative and supportive shaft so that it may be rotated or turned or swung around the shaft in two opposite directions, or the directions of arrows A1 and B1 in FIG. 2 or arrows A2 and B2 in FIG. 6.

An oblong leaf spring 33 or the like is arranged as urging means in the inside of the lever 30. The leaf spring 33 is bent along an edge thereof and the bent section of the leaf spring 33 is secured in position as it is pinched by a holding means 21b fitted to the main body 21, while the opposite edge of the leaf spring 33 is secured in position as it is made to abut a spring bearing 34 of which other end arranged near the front end of the lever main body 31 of the lever 30. With this arrangement, the lever main body 31 of the lever 30 is urged by the leaf spring 33 in the direction of arrow B2.

The sleeve 32 integrally arranged at an end of the lever 30, or at the top end in FIG. 2, has a closed end portion of which end edge portion 32b is closed. And location of the fixed piston 23 is an opening 32a. The wall portion 32c surrounding the opening 32a defines cylinder-shaped internal spaces S1, S2 that are open only at an end. While the internal spaces S1, S2 is a single space, the single space is divided into an open space S2 that is open to the atmosphere and a closed space S1 that is defined by the (inner) wall section 32c and the inside of the end section 32b of the sleeve 32 when the fixed piston 23 is inserted into the sleeve 32 through the opening 32a.

A pressure regulating means 37 is fitted to the outer surface of the closed end section 32b which is the closed end of the sleeve 32 to make the pressure of the internal space S1 adjustable. A number of projections may be arranged on the inner wall of the closed end section 32b of the sleeve 32 to such a height that they may not hit the fixed piston 23 for the purpose of reducing the volume of air in the internal space S1 and boosting the negative pressure in the inside when the breast pump is in operation. At least the inner wall 32d of the sleeve 32 is made to show a profile that substantially agree with the locus of the rotary motion of the top end and that of the bottom end of the lever 30. Thus, the sleeve 32 shows a profile that substantially agree with the locus of the rotary motion of the top end and that of the bottom end of the lever 30, the lever 30 can be moved to rotate smoothly and easily.

On the other hand, the fixed piston 23 arranged at the side of the main body 21 has a profile that allows the piston to tightly contact the inner wall of the sleeve 32 and a ring-shaped packing 23a that is an elastic member and operates as piston ring is fitted to the flange-shaped end of the fixed piston. At least a part of the outer periphery of the packing 23a is inclined toward the lever 30 (left side in FIG. 2) so as to reduce its dimensions. Due to the inclination, as the outermost periphery of the elastic packing 23a abuts the entire periphery of the inner wall 23d, the airtight closure is maintained and the lever 30 can be operated lightly.

As shown in FIG. 1, the wall of the sleeve 32 of the lever 30 is partly indented at a position near the top end of the sleeve 32 to produce a leak section 38. This section is located at the end of the sleeve 32 in terms of the relative reciprocating motion of the fixed piston 23, which will be described hereinafter. This portion functions in a state when negative pressure is maximized.

Thus, for an operation of breaking negative pressure, the inside of the lever 30 is made partly open to the atmosphere by way of the leak section 38 only when the fixed piston 23 is moved to the leak section 38 so that the lever 30 can be operated easily without exerting extremely large force. It may be so arranged that the entire peripheral edge of the fixed piston 23 or an upper region thereof simultaneously comes out of the sleeve 32 for an operation of breaking negative pressure.

As will be described hereinafter, any increase of negative pressure can be rapidly reduced to make pulsative change smoothly by allowing air to leak after the lever main body 31 is moved toward the bottle 41 to consequently increase negative pressure.

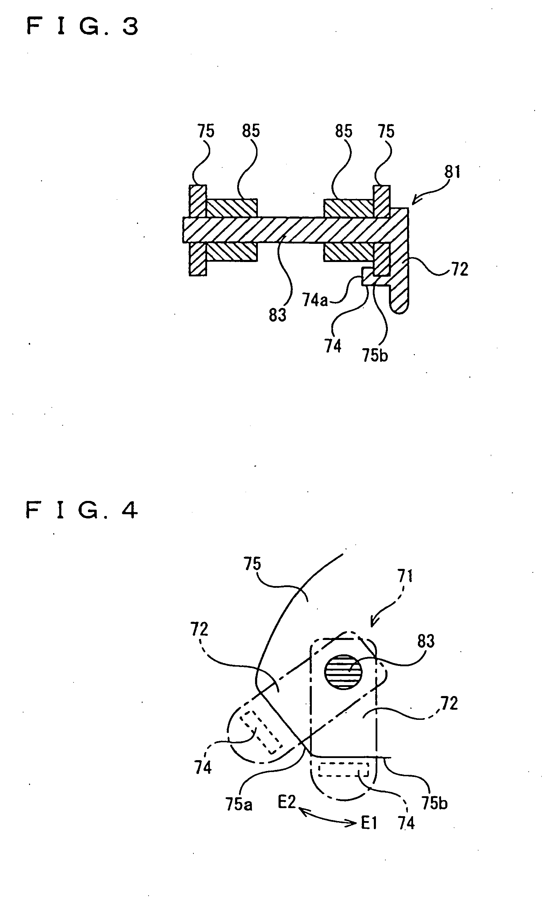

Now, the arrangement of securing the lever 30 to the main body 21 by means of a rotative and supportive shaft will be described by referring to FIGS. 3 and 4.

FIG. 3 shows an example of a structure that can be used to rigidly secure the lever 30 of the breast pump 20 to the detachable connecting portion 25.

The detachable connecting portion 25 is provided as integral portion thereof with a pair of bearing portions 85, 85 for the main body 21. On the other hand, the lever 30 is provided with a pair of bearing portions 75, 75 that are located outside of the width between bearing portions 85 and 85.

An oblong shaft member 81 is provided with a base portion 72 and a projection or ridge 74. The shaft member 81 has a shaft 83 that is inserted in such a way it penetrate the lever side bearings 75, 75 and the detachable connecting portion side bearings 85, 85. A holding pawl 74a is arranged at the front end of the projection or ridge 74 in order to prevent the shaft member 81 from inadvertently coming off from the bearing portion.

As shown in FIG. 4, the bearing portion 75 of the lever 30 is provided with a sloped surface 75a and a horizontal engaging surface 75b continued to the sloped surface 75a at a lower portion of the bearing portion 75.

Then, the bearing portion 75 of the lever 30 is placed on the corresponding bearing portion (not shown) of the main body 21 and the shaft 83 of the shaft member 81 is driven to run through the shaft holes 76, 76 of the bearing portions 75 from the both outside of each bearing portion 75.

As showing in FIG. 4, firstly, the shaft member 81 is inclined. Then, as the shaft 83 is driven to run through the shaft holes 76 and the shaft member 81 is rotated around the shaft 83 in the direction of arrow E1, the projection 74 comes to be engaged with or held by the horizontal engaging surface 75b.

As a result, the lever 30 is secured to the main body 21. Conversely, to remove the lever 30 from the main body 21, the shaft member 81 is rotated in the direction of arrow E2 in FIG. 4. Then, the projection 74 is released from the horizontal engaging surface 75b. As a result, the lever 30 can be removed from the main body 21.

In this way, the lever 30 is securely held only by the shaft member 81 be inserted into the shaft holes 76 and by the operation for mutual engagement. Therefore, the assembling operation is very simple and easy. Additionally, the lever 30 can be removed from the main body 21 simply by removing the shaft member 81 from the shaft holes 76 so that assembling and disassembling is easy. However, the lever cannot be removed from the main body 21 when the shaft member 81 is inserted by and engaged with the bearing portion 75. In short, while the lever 30 can be easily fitted to and removed from the main body 21, it is effectively prevented from inadvertently coming off from the main body 21.

FIG. 2 shows that the embodiment of breast pump 20 having the above described configuration in an unused or mint state. It is operated in a manner as described below.

Referring to FIG. 2, the lever 30 is fitted to the main body 21 and the bottle 41 is fitted to the main body 21 by way of the detachable connecting portion 25.

Under this condition, the sleeve 32 of the lever 30 has been moved in the direction of arrow A2 by the leaf spring 33 so that the fixed piston 23 is located deep in the inside of the sleeve 32.

Then, the user applies the suction portion 22 to the breast by way of the opening thereof so that the breast may be surrounded by the suction portion 22. Then, the user applies the thumb of one of his or her hands to the bottle 41 and all or some of the fingers of the hand to the lever main body 31 of the lever 30 (not shown) . Under this condition, the user closes the hand to operate the lever main body 31. Then, the lever 30 turns around the shaft in the direction of arrow A1 and also in the direction of arrow B1 as shown in FIG. 1 against the urging force of the leaf spring 33. During this process, the fixed piston 23 is moved relatively in the direction of arrow D to increase the volume of the internal space S1 of the sleeve 32. As a result, the negative pressure in the inside of the internal space S1 of the sleeve 32 is increased and the increased negative pressure is made to propagate to the suction portion vent passage 22a by way of the vent passage 27 and the cell 26a. Thus, negative pressure prevails in the inside of the suction portion 22 to suck mother's milk from the breast and the sucked mother's milk is driven to pass through the suction portion vent passage 22a and stored in the cell 26a. At this time, by convex portion 28a of the buffer 28 arranged in the inside of the suction portion 22, the breast drawn by the negative pressure in the inside of the suction portion 22 so as to abut, is pressed and provided an effect similar to that of massage.

At this time, since negative pressure prevails in the inside of the cell 26a, the oppositely disposed lateral walls 26b, 26c of the cellular valve 26 are drawn upward to close the slit 26d.

Then, as the user grasps the lever main body 31 in the direction of arrow BB until it comes to abut the outer edge of the bottle 41, the fixed piston 23 is moved relatively in the direction of arrow D. Eventually, the fixed piston 23 comes to the end of the relative movement and air infiltrates into the internal space S1 of the sleeve 32 through the leak portion 38. As a result, the negative pressure in the internal space S1 is quickly reduced and the internal pressure of the cell 26a that is held in communication with the internal space S1 by way of the vent passage 27 also comes close to the atmospheric pressure to decrease the effect of drawing the oppositely disposed lateral walls 26b, 26c of the cellular valve 26 upward. Then, the slit 26d constructed at the lower ends of the lateral walls 26b, 26c is opened by the weight of the mother's milk stored in the cell 26a and the milk stored in the cell 26a falls into the inside portion S3 of the bottle 41.

As a result of this process, the sucking pressure of the suction portion 22 applied to the breast of user falls.

Then, the lever 30 is returned to the position shown in FIG. 2 by the urging force of the leaf spring 33. Thus, operation of sucking milk can be continued by the user repeating the above process.

Therefore, with the embodiment of breast pump 20 according to the invention, the user is not required to handle the breast pump with his or her two hands unlike the conventional breast pump described above by referring to FIG. 3. So, the user only needs to use his or her dominant hand to handle the breast pump 20. Thus, the operation is simple and easy.

Additionally, the embodiment of breast pump 20 does not involve the use of a conventional long air pipe and the like, which is cumbersome and difficult to handle. So, the embodiment of breast pump 20 is remarkably superior in storage and portability.

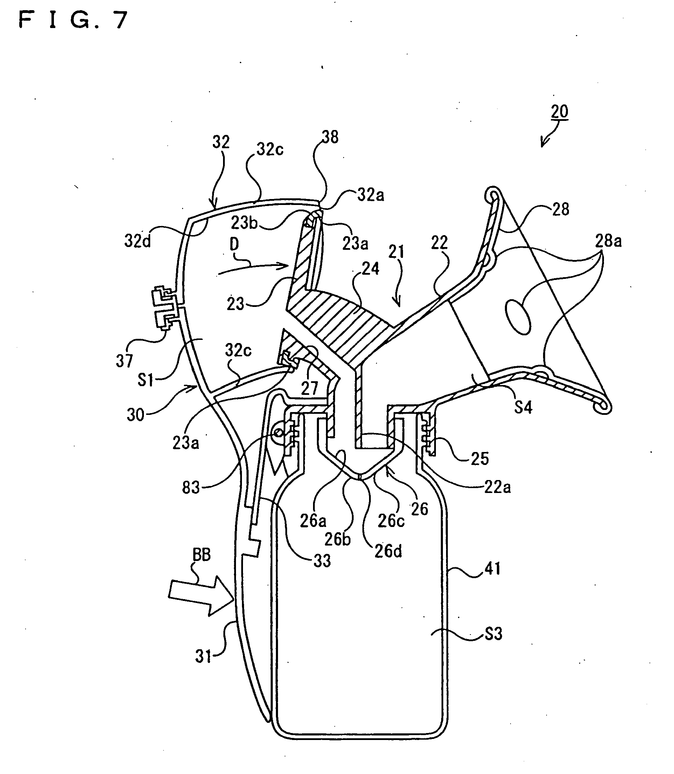

As described above and illustrated in FIGS. 2, 6 and 7, once the lever 30 is fitted to the main body 21, engagement of the rotative and supportive shaft structure of the lever 30 with the main body 21 is not detached at any changeful position in the state that the bottle 41 is fitted to the main body 21. In other words, once the lever 30 is fitted to the main body 21, the status that main body 21 is fitted to the bottle 41, the situations that the user can proceed under the operation of sucking milk, the lever is constructed not to come off from the main body 21.

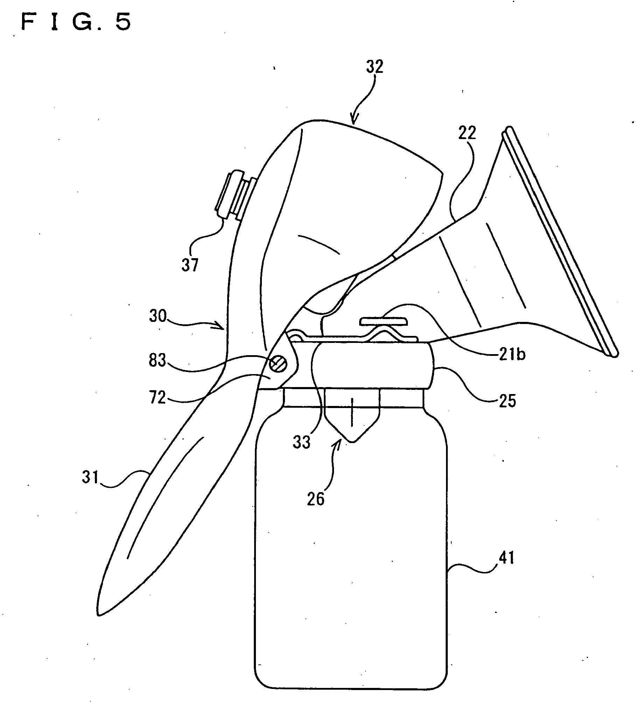

Thus, the lever 30 is securely held to the main body 21 only by the shaft member 81 be inserted into the shaft hole 76 and by operation for mutual engagement. Therefore, the assembling is very simple and easy. Additionally, the lever 30 can be removed simply by removing the shaft member 81 from the shaft hole 76 so that the assembling and disassembling is easy. Furthermore, in the breast pump 20, the leaf spring 33 as urging means is held at an end thereof by the holding means 21b of the main body 21 as shown in FIG. 5 and made at the other end thereof to abut the spring bearing 34 arranged near the front end of the lever main body 31 of the lever 30 in a manner as described above. Therefore, when the lever 30 is removed from the main body 21, it can be taken out simultaneously with ease and cleaned. When the urging means is a leaf spring 33 that is made of metal, stainless steel (SUS304) in particular, it is superior in durability and can surely rebound. Additionally, the leaf spring 33 can be taken out to sterilize the remaining components of the breast pump 20 by means of a chemical such as sodium hypophosphite and the like. In this case, this operation is conducted safely because the leaf spring 33 cannot be taken out without removing the lever 30 from the main body 21.

Furthermore, in the breast pump 20, the sleeve 32 is integrally formed at an end of the lever 30. Thus, the opening for inserting the fixed piston 23 and the sleeve 32 of which internal space are exposed can be cleaned by only removing the lever 30, so, the space for generating negative pressure has a simple configuration and can be cleaned with ease. Additionally, once the lever 30 is removed, the fixed piston 23 from which the sleeve 32 has been moved away comes to be exposed on the main body 21 so that it can also be cleaned with ease. In this case, since the sleeve 32 is made movable while the piston is fixed, the piston rod 24 of the fixed piston 23 does not require any junction such as link. So, the piston rod 24 is free from parts where dirt of dregs and the like of mother' s milk and the like would easily adhere and is difficult to remove so that it can be cleaned with ease and held in a highly hygienic condition.

In short, the mechanism for generating negative pressure can be exposed for cleaning by a simple operation of removing the lever 30 from the main body 21.

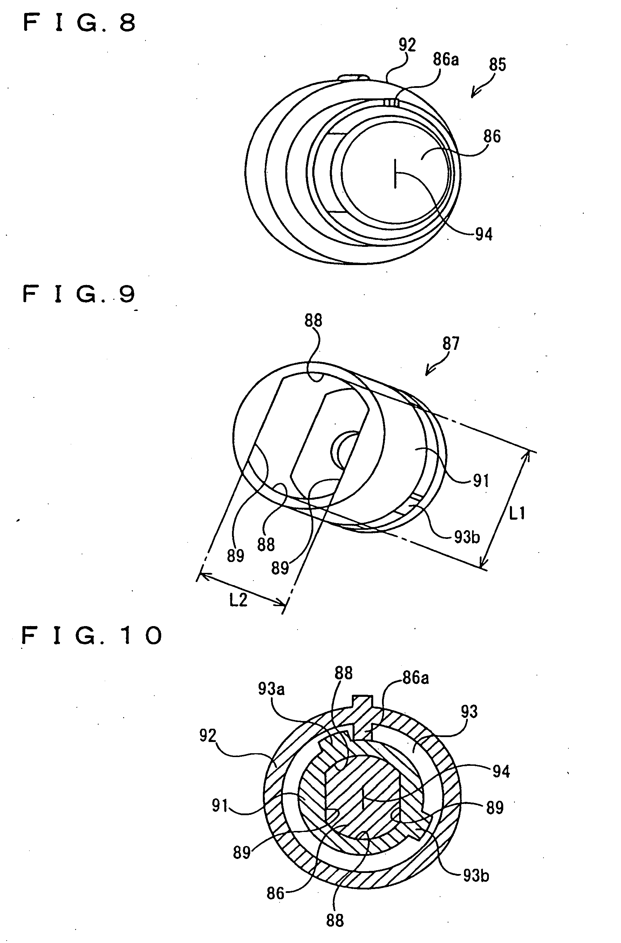

FIGS. 8 through 12 illustrate the valve structure of the pressure regulating means 37 arranged on the outer surface of the sleeve 32 of the breast pump 20.

FIG. 8 shows the valve main body 85 that is part of the pressure regulating means 37 and FIG. 9 shows the valve box portion 87 that is to be combined with the valve main body 85.

The valve box 91 of the valve box portion 87 is a bottomed cylinder having two pairs of oppositely disposed inner walls 88, 88 and 89, 89 in the inside. The pair of oppositely disposed inner walls 88, 88 are separated from each other by distance L1, which is greater than distance L2 by which the other pair of oppositely disposed inner walls 89, 89 are separated from each other. L1 is substantially equal to or slightly greater than the diameter of the circular valve body 86. The distance L2 is smaller than the diameter of the valve body 86.

As shown in FIG. 8, the valve main body 85 that is housed in the valve box 91 has a valve body 86 at the front end of a substantially flat and cylindrical base 92 that is made of a hard material. The valve body 86 has a circular outer periphery and a slit 94 located at the center thereof. At least a part of the valve body 86 where the slit 94 is formed is made of an elastic material such as rubber.

As for the situation that, the valve body 86 is housed in the valve box 91 shown in FIG. 9. FIG. 10 is a cross sectional view of the valve body 86 and the abstract of situation of a stopper.

Referring to FIG. 10, the stopper 86a projecting inward is arranged on the inner periphery of the base section 92. On the other hand, the valve case 91 is provided at a base section thereof with a guide groove 93 for receiving the stopper 86a. The guide groove 93 contains therein a first positioning portion 93a projecting outward and a second positioning section 93b spaced from the first positioning section 93a by 1/4 of the circumference or more.

As seen from FIG. 10, when the stopper 86a abuts the first positioning section 93a, the valve main body 86 is housed in the valve box 91 in such a way that the slit 94 locates so as to connect the inner walls 88, 88. So, the longitudinal direction of the slit 94 agrees with that of the longer distance L1 so that the slit 94 is closed. Additionally, since the distance L2 is small relative to the disk-shaped valve body 86, the slit 94 is pushed and pressed to the direction to be pushed. Thus, the pressure regulating means 37 is closed and the inner space S1 is isolated from the atmosphere.

On the other hand, as the valve main body 86 is turned in the valve case 91 and the base portion 93 is relatively turned in the direction indicated by an arrow, the distance that contains the longitudinal direction of the slit 94 is gradually reduced and the opposite ends 94a, 94 of the slit 94 which was crushed the slit becomes open to allow air to pass through it slightly. As a result, the negative pressure in the internal space S1 of the sleeve 32 is slightly reduced.

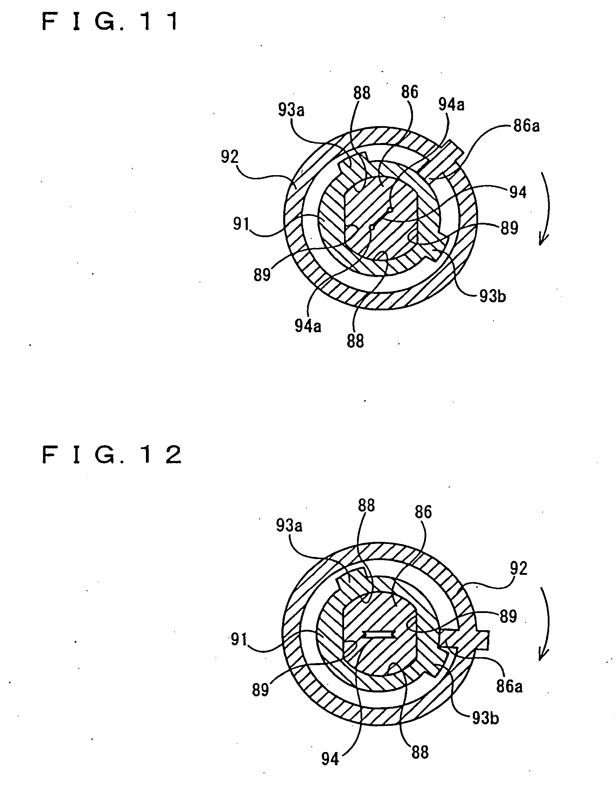

Then, as shown in FIG. 11, as the valve main body 86 is turned further in the valve box 91 in the direction indicated by the arrow until the stopper 86a abuts the second positioning portion 93b, the distance that contains longitudinal direction of the slit 94 is further reduced. Since the longitudinal direction of the slit 94 is in the direction of the shorter distance L2, the slit 94 becomes fully open. As a result, air passes at an enhanced rate to remarkably reduce the negative pressure in the internal space S1 of sleeve 32.

By the way, the present invention is by no means limited to the above described embodiment.

For instance, the outer surface of the lever main body of the lever 30 may be provided with anti-slippery undulations or a finger hook so that the lever 30 may be handled with ease.

After the above described embodiment, or example of modification, some of the components may be omitted if needed or combined with other components as set out in the claims.

### INDUSTRIAL APPLICABILITY

As described above in detail, the present invention provide a manual breast pump that can be conveniently operated by hand.

## Claims

1. A manual breast pump (20) comprising:
a breast pump main body (21) having a substantially conical suction portion (22) to be applied to a breast, and a detachable connecting portion (25) detachably fitted to a bottle (41) to communicate with the bottle; and
a lever (30) with a long shape extending in a direction and held by a shaft (83) on the breast pump main body at a middle part thereof by means of a rotative and supportive shaft means (75, 85, 81), the lever being rotatable in opposite directions around the shaft,
wherein the suction portion communicates with a predetermined internal space (S1) that contains a fixed piston (23)
wherein movement of an end of said lever (30) causes the lever to rotate and results in the relative reciprocating motion of the fixed piston (23), the motion of the piston generating negative pressure in said internal space (S1) through a change of the internal space (S1) to suck milk from said suction portion and make the sucked mother's milk drop into said bottle (41); and
a negative pressure generating means comprising a vent passage (27) communicating with said suction portion (22), the fixed piston (23) arranged at an end of the vent passage (27), and a sleeve (32) arranged integrally at an end of said lever (30), into which said fixed piston (23) of said breast pump main body (21) is inserted to communicate with said vent passage (27), said inserted fixed piston (23) being adapted to slide in the inside of said sleeve (32) when said lever (30) is rotated so as to generate negative pressure by rotating of the lever (30) in the space (S1) defined by the inner surface of said sleeve (32) and a face of said fixed piston (23).

2. The breast pump (20) as claimed in Claim 1, wherein the rotative and supportive shaft means (75, 85, 81) has a bearing portion (75) arranged at the side of said lever (30) and a bearing portion (85) arranged at the side of said breast pump main body; and
the shaft (83) is supported by a shaft member (81) and is insertable through the shaft holes (76) of the bearing portions laid one on the other; and
the shaft member (81) is provided with an engaging means (74) for engagement with any of said bearing sections.

3. The breast pump (20) as claimed in either one of claim 1 or claim 2, wherein at least the inner wall (32d) of said sleeve (32) has a curved profile that agrees with the locus of the rotary motion of an end of the lever (30).

4. The breast pump (20) as claimed in any one of Claims 1 to 3, wherein the outer periphery (23a) of the fixed piston (23) is elastic and inclined toward the lever (30) to reduce its dimensions.

5. The breast pump (20) as claimed in any one of Claims 1 to 4, wherein when the non-sleeve end (31) of the lever (30) is moved closer to said bottle (41) the fixed piston (23) no longer makes an air-tight contact with the inner wall (32d) of the sleeve (32) at the end of said sleeve (32).

6. The breast pump (20) as claimed in any one of Claim 1 to 5, wherein the sleeve (32) is provided on the outer surface (32b) of its closed end with a pressure regulating means (37).

7. The breast pump (20) as claimed in Claim 6, wherein the pressure regulating means (37) is arranged on the outer surface (32b) of the closed end of the sleeve (32), the pressure regulating means (37) comprising;
a valve box (91) having at least two pairs of oppositely disposed inner walls (88, 89), the distance L1 separating one of the pairs of oppositely disposed inner walls being greater than the distance L2 separating the other pair of oppositely disposed inner walls; and
a valve body (86) made of an elastic material and having a substantially circular outer periphery and a slit (94) for allowing air to pass through;
wherein the opening of said slit (94) is adjustable by inserting said valve body in said valve box and rotating the valve body in said valve box.

## Patentansprüche

1. Manuelle Brustpumpe (20), die Folgendes umfasst:
einen Brustpumpenhauptkörper (21), der einen im Wesentlichen konusförmigen Saugabschnitt (22), der an die Brust anzulegen ist, und einen abnehmbaren Verbindungsabschnitt (25) aufweist, der abnehmbar an eine Flasche (41) angepasst ist, um mit der Flasche zu kommunizieren und
einen Hebel (30), mit einer länglichen Form, der sich in eine Richtung erstreckt und durch eine Welle (83) durch ein rotierbares und stützendes Wellenmittel (75, 85, 81) an einem Mittelteil des Brustpumpenhauptkörpers gehalten wird, wobei der Hebel in entgegengesetzte Richtungen um die Welle rotierbar ist,
wobei der Saugabschnitt mit einem vorbestimmten Innenraum (S1) kommuniziert, der einen fixierten Kolben (23) enthält,
wobei die Bewegung eines Endes des Hebels (30) bewirkt, dass sich der Hebel dreht und zu einer relativen Hin- und Herbewegung des fixierten Kolbens (23) führt, wobei die Bewegung des Kolbens durch eine Veränderung des Innenraums (S1) Unterdruck in dem Innenraum (S1) erzeugt, um Milch aus dem Saugabschnitt zu saugen und zu bewirken, dass die abgesaugte Muttermilch in die Flasche (41) tropft; und
ein Unterdruckerzeugungsmittel, das Folgendes umfasst: einen Entlüftungsdurchlass (27), der mit dem Saugabschnitt (22) kommuniziert, wobei der fixierte Kolben (23) an einem Ende des Entlüftungsdurchlasses (27) angeordnet ist, und eine Manschette (32), die einstückig an einem Ende des Hebels (30) ausgebildet ist und in die der fixierte Kolben (23) des Brustpumpenhauptkörpers (21) eingeführt ist, um mit dem Entlüftungsdurchlass (27) zu kommunizieren, wobei der eingeführte fixierte Kolben (23) geeignet ist, um in das Innere der Manschette (32) zu gleiten, wenn der Hebel (30) gedreht wird, um durch die Drehung des Hebels (30) in dem durch die Innenoberfläche der Manschette (32) und einer Fläche des fixierten Kolbens (23) definierten Raum (S1) Unterdruck zu erzeugen.

2. Brustpumpe (20) nach Anspruch 1, worin
das rotierbare und lagernde Wellenmittel (75, 85, 81) einen Lagerabschnitt (75), der an der Seite des Hebels (30) angeordnet ist, und einen Lagerabschnitt (85) aufweist, der an der Seite des Brustpumpenhauptkörpers angeordnet ist, und
die Welle (83) durch ein Wellenelement (81) gelagert wird und durch die Wellenlöcher (76) der übereinanderliegenden Lagerabschnitte einführbar ist; und
das Wellenelement (81) mit einem Eingriffselement (74) bereitgestellt ist, um mit einem der Lagerabschnitte in Eingriff zu gelangen.

3. Brustpumpe (20) nach Anspruch 1 oder 2, worin zumindest die Innenwand (32d) der Manschette (32) ein gekrümmtes Profil aufweist, das mit der Ortskurve der Rotationsbewegung am Ende des Hebels (30) übereinstimmt.

4. Brustpumpe (20) nach einem der Ansprüche 1 bis 3, worin der Außenumfang (23a) des fixierten Kolbens (23) elastisch und in Richtung des Hebels (30) geneigt ist, um seine Dimensionen zu reduzieren.

5. Brustpumpe (20) nach einem der Ansprüche 1 bis 4, worin der fixierte Kolben (23), wenn das Ende (31) des Hebels (30), das von der Manschette abgewandt ist, näher an die Flasche (41) bewegt wird, am Ende der Manschette (32) nicht mehr in luftdichtem Kontakt mit der Innenwand (32d) der Manschette (32) steht.

6. Brustpumpe (20) nach einem der Ansprüche 1 bis 5, worin die Manschette (32) an der Außenoberfläche (32b) ihres geschlossenen Endes mit einem Druckregulierungsmittel (37) versehen ist.

7. Brustpumpe (20) nach Anspruch 6, worin das Druckregulierungsmittel (37) an der Außenoberfläche (32b) des geschlossenen Endes der Manschette (32) angeordnet ist, wobei das Druckregulierungsmittel (37) Folgendes umfasst:
ein Ventilgehäuse (91) mit zumindest zwei Paar einander gegenüberliegend angeordneter Innenwände (88, 89), wobei der Abstand L1 zwischen einem der Paare einander gegenüberliegend angeordneter Innenwände größer ist als der Abstand L2 zwischen dem anderen Paar einander gegenüberliegend angeordneter Innenwände und
einen Ventilkörper (86) aus einem elastischen Material mit einem im Wesentlichen kreisförmigen Außenumfang und einem Spalt (94), durch den Luft hindurchtreten kann;
wobei die Öffnung des Spalts (94) anpassbar ist, indem der Ventilkörper in das Ventilgehäuse eingeführt wird und darin rotiert wird.

## Revendications

1. Tire-lait manuel (20) comprenant:
un corps principal de tire-lait (21) ayant une portion de succion sensiblement conique (22) à appliquer à un sein, et une portion de connection amovible (25) montée amoviblement sur une bouteille (41) pour communiquer avec la bouteille; et
un levier (30) d'une forme longue s'étendant dans une direction et retenu par un arbre (83) sur le corps principal de tire-lait à sa partie médiane par un moyen d'arbre de rotation et de support (75, 85, 81), le levier pouvant tourner dans des directions opposées autour de l'arbre,
où la portion de succion communique avec un espace interne prédéterminé (S1) qui contient un piston fixe (23)
où le mouvement d'une extrémité dudit levier (30) amène le levier à tourner et se traduit par un mouvement de va-et-vient relatif du piston fixe (23), le mouvement du piston produisant une pression négative dans ledit espace interne (S1) par un changement de l'espace interne (S1) pour aspirer le lait de ladite portion de succion et pour amener le lait maternel aspiré à tomber dans ladite bouteille (41); et
un moyen de génération de pression négative comprenant un passage d'aération (27) communiquant avec ladite portion de succion (22), le piston fixe (23) étant agencé à une extrémité du passage d'aération (27), et un manchon (32) agencé intégralement à une extrémité dudit levier (30), dans lequel ledit piston fixe (23) dudit corps principal de tire-lait (21) est inséré pour communiquer avec ledit passage d'aération (27), ledit piston fixe inséré (23) étant apte à coulisser dans l'intérieur dudit manchon (32) lorsque ledit levier (30) est amené à tourner de manière à produire une pression négative en faisant tourner le levier (30) dans l'espace (S1) défini par la surface intérieure dudit manchon (32) et une face dudit piston fixe (23).

2. Tire-lait (20) selon la revendication 1, dans lequel le moyen d'arbre de rotation et de support (75, 85, 81) possède une portion de support (75) agencée au côté dudit levier (30) et une portion de support (85) agencée au côté dudit corps principal de tir-lait; et
l'arbre (83) est supporté par un élément d'arbre (81) et peut être inséré à travers les trous d'arbre (76) de portions de support placées l'une au-dessus de l'autre; et
l'élément d'arbre (81) présente un moyen d'engagement (74) pour la mise en prise avec l'une quelconque desdites sections de support.

3. Tir-lait (20) selon l'une quelconque de la revendication 1 ou de la revendication 2, dans lequel au moins la paroi intérieure (32d) dudit manchon (32) possède un profil courbé qui coïncide avec le lieu du mouvement de rotation d'une extrémité du levier (30).

4. Tir-lait (20) selon l'une quelconque des revendications 1 à 3, dans lequel la périphérie extérieure (23a) du piston fixe (23) est élastique et est inclinée vers le levier (30) pour réduire ses dimensions.

5. Tir-lait (20) selon l'une quelconque des revendications 1 à 4, dans lequel, lorsque l'extrémité non-manchon (31) du levier (30) est rapprochée de ladite bouteille (41), le piston fixe (23) n'établit plus de contact étanche à l'air avec la paroi intérieure (32d) du manchon (32) à l'extrémité dudit manchon (32).

6. Tir-lait (20) selon l'une quelconque des revendications 1 à 5, dans lequel le manchon (32) est réalisé sur la surface extérieure (32b) de son extrémité fermée avec un moyen de régulation de pression (37).

7. Tir-lait (20) selon la revendication 6, dans lequel le moyen de régulation de pression (37) est agencé sur la surface extérieure (32b) de l'extrémité fermée du manchon (32), le moyen de régulation de pression (37) comprenant:
une boîte de vanne (91) ayant au moins deux paires de parois intérieures disposées d'une manière opposée (88, 89), la distance L1 séparant une des paires de parois intérieures disposées d'une manière opposée étant plus grande que la distance L2 séparant l'autre paire de parois intérieures disposées d'une manière opposée; et
un corps de vanne (86) réalisé en un matériau élastique et ayant une périphérie extérieure sensiblement circulaire et une fente (94) pour permettre le passage d'air à travers celle-ci;
où l'ouverture de ladite fente (94) est ajustable en insérant ledit corps de vanne dans ladite boîte de vanne et en faisant tourner le corps de vanne dans ladite boîte de vanne.
